(19) Europäisches Patentamt
European Patent Office
Office européen des brevets

(11) Publication number: 0 112 184 B1

(12) # EUROPEAN PATENT SPECIFICATION

(45) Date of publication of patent specification: 26.04.89

(51) Int. Cl.⁴: C 12 P 21/02, A 61 K 37/02, C 07 K 9/00 // (C12P21/02, C12R1:365, C12P21:04, C12R1:365)

(21) Application number: 83307676.3

(22) Date of filing: 16.12.83

(54) **Antibiotic A51568 factors A and B.**

(30) Priority: 20.12.82 US 450880

(43) Date of publication of application:
27.06.84 Bulletin 84/26

(45) Publication of the grant of the patent:
26.04.89 Bulletin 89/17

(84) Designated Contracting States:
AT BE CH DE FR IT LI LU NL SE

(56) References cited:
EP-A-0 055 069
EP-A-0 055 070

THE JOURNAL OF ANTIBIOTICS, vol. 37, no. 8, August 1984, TOKYO, (JP), Pages 917-919. A. HUNT et al.: "N-Demethylvancomycin".

(73) Proprietor: ELI LILLY AND COMPANY
307, East McCarty Street
Indianapolis Indiana 46285 (US)

(72) Inventor: Hoehn, Marvin Martin
4975 East 79th Street
Indianapolis Indiana 46250 (US)
Inventor: Marconi, Gary G.
8616 Meadow Vista Drive
Indianapolis Indiana 46217 (US)

(74) Representative: Hudson, Christopher Mark et al
Erl Wood Manor
Windlesham Surrey GU20 6PH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 112 184 B1

**Description**

This invention relates to the antibiotics designated herein as A51568, Factors A and B, which are produced by culturing a hitherto undescribed microorganism, *Nocardia orientalis* NRRL 15232, or an A51568-producing mutant or variant thereof, under submerged aerobic fermentation conditions. *Nocardia orientalis* NRRL 15232 normally produces a mixture of A51568 factors A and B in a ratio of about 95 to 5. These antibiotics inhibit the growth of pathogenic microorganisms, in particular, those within the gram-positive genera *Staphylococcus* and *Streptococcus*, which are resistant to penicillin.

A great many microorganisms are pathogenic and are causative agents of disease states in both humans and animals. Over the years, a great number of antibiotics which are active against pathogenic microorganisms have been developed. However, there is still a need to find agents which are more effective against these pathogenic microorganisms in order to more successfully treat the diseases caused by the microorganisms in humans or animals.

Antibiotic A51568, factors A and B, belong to the glycopeptide family of antibiotics, and are gram-positive antimicrobial agents.

Glycopeptide antibiotics already known in the art include, *inter alia*, vancomycin, McCormick *et al.*, U.S. Patent No. 3,067,099 (December 4, 1962), the structure of vancomycin being reported by Williamson *et al., J. Am. Chem. Soc. 103,* 6580—6585 (1981); actaplanin (antibiotic A—4696), Hamill *et al.*, U.S. Patent No. 3,952,095 (April 20, 1976), a portion of the structure of actaplanin being reported by Debono, U.S. Patent No. 4,322,343 (March 30, 1982); ristocetin, British Patent No. 765,886 (1957), the structure of ristocetin A, one factor of the ristocetin complex, being reported by Kalman *et al., J. Am. Chem. Soc. 102,* 897—905 (1980); and avoparcin, Kunstmann *et al.,* U.S. Patent No. 3,338,786 (August 29, 1967), the structure of avoparcin being described by Ellestad *et al., J. Am. Chem. Soc. 103,* 6522—6524 (1981).

Specifically, this invention provides an isolated antibiotic having the structural formula (I):

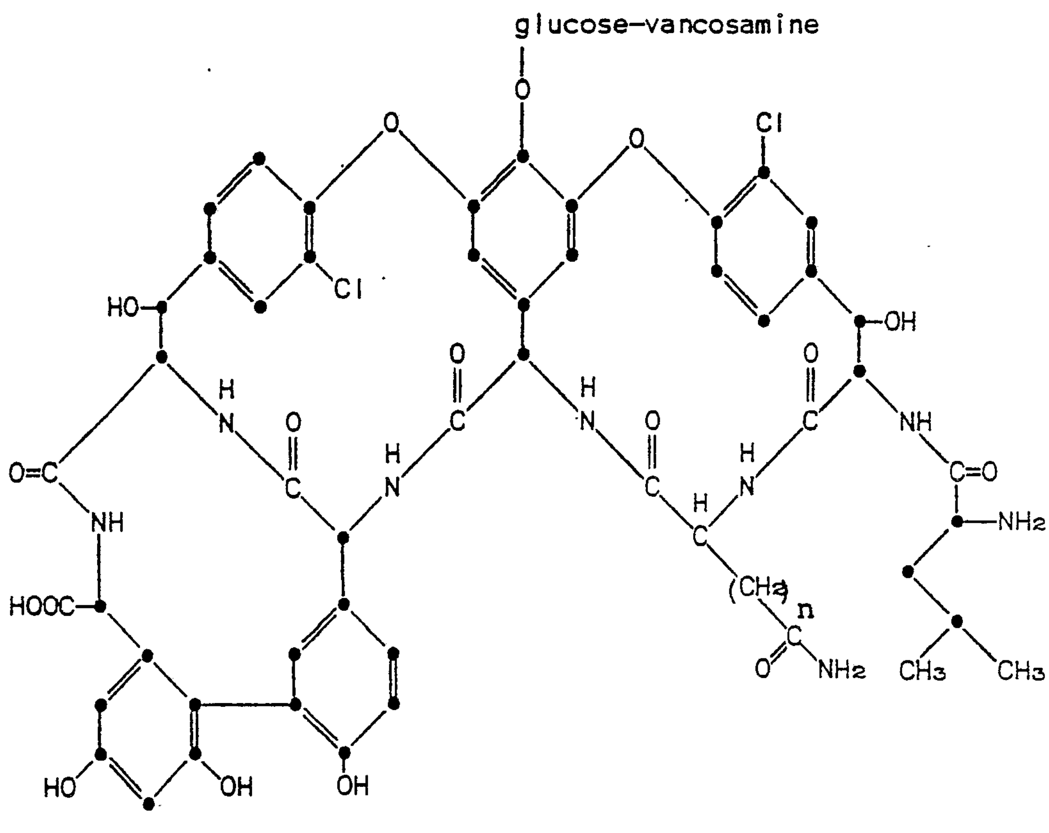

wherein n is 1 or 2, or a pharmaceutically acceptable salt thereof, or an antibiotic mixture comprising an antibiotic of formula (I) or a pharmaceutically acceptable salt thereof as the major component. The antibiotic of formula (I) wherein n is 1 has been designated A—51568 factor A. The antibiotic of formula (I) wherein n is 2 has been designated A—51568 factor B.

Antibiotic A51568 factor A is a white, amorphous solid. Elemental analysis of antibiotic A51568 factor A indicates the following approximate percentage composition: 50.63 percent carbon, 5.07 percent hydrogen, 8.36 percent nitrogen, 8.19 percent chlorine, and 25.16 percent oxygen. The antibiotic has a molecular weight of about 1435, as determined by fast-atom-bombardment mass spectrometry.

The proton nuclear magnetic resonance spectrum of antibiotic A51568 factor A was determined in

dimethylsulfoxide at 60°C and 360 MHz. The several six-membered rings of the structural formula are identified by letters of the alphabet, as indicated in the following formula:

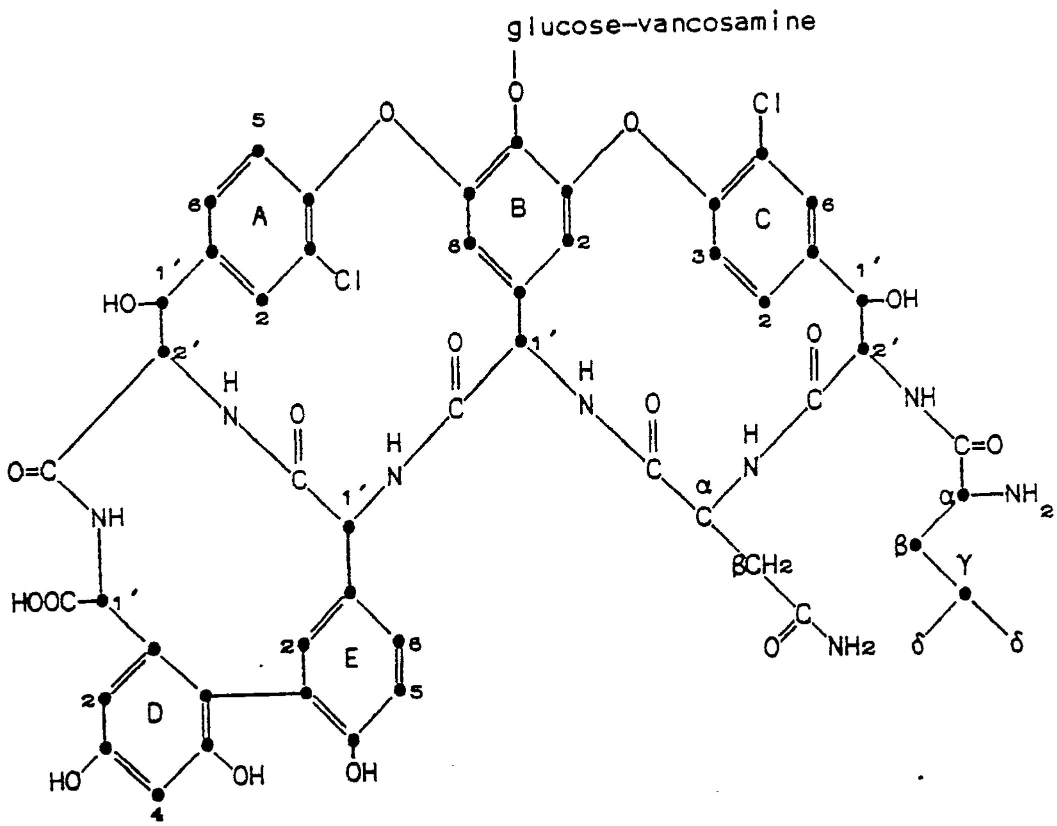

The table of Chemical Shifts, in ppm, follows in Table 1.

Table 1

Chemical Shifts for A51568 factor A (60°C)

| Assignment Ring A | Chem. Shift | Assignment Asparagine | Chem. Shift |
|---|---|---|---|
| -NH | 6.54 | -NH | 6.61 |
| -2' | 4.19 | -α | 4.32 |
| -1' | 5.14 | -β's | 2.56 & |
| -OH | 5.86 | | 2.14 |
| -2 | 7.86 | $-NH_2$ | 7.29 & |
| -5 | 7.30 | | 6.83 |
| -6 | 7.47 | | |
| | | **Leucine** | |
| **Ring B** | | $-NH_2$ | ____* |
| -NH | 8.24 | -α | 3.70 |
| -1' | 5.72 | -β's | 1.65 & |
| -2 | 5.63 | | 1.49 |
| -6 | 5.22 | -γ | 1.76 |
| | | -δ's | 0.93 & |
| | | | 0.90 |
| **Ring C** | | **Glucose** | |
| -NH | ____* | -1 | 5.33 |
| -2' | 4.78 | -2 | 3.58 |
| -1' | 5.18 | -3 | 3.49 |
| -OH | 5.74 | -4 | 3.29 |
| -2 | 7.66 | -5 | ____* |
| -3 | 7.18 | -6 | 3.70 & |
| -6 | 7.42 | | 3.54 |
| **Ring D** | | **Vancosamine** | |
| -NH | 8.45 | -1 | 5.30 |
| -1' | 4.47 | -2 | 1.93 & |
| -2 | 6.26 | | 1.77 |
| -4 | 6.42 | $(3-CH_3)$ | 1.37 |

Table 1 cont'd.

Chemical Shifts for A51568 factor A

| Assignment Ring E | Chem. Shift | Assignment Vancosamine | Chem. Shift |
|---|---|---|---|
| -NH | 8.54 | 4 | ____* |
| -1' | 4.47 | 5 | 4.67 |
| -2 | 7.17 | (5-$CH_3$) | 1.09 |
| -5 | 6.72 | | |
| -6 | 6.79 | | |

*Not assigned

The proton nuclear magnetic resonance spectrum of antibiotic A51568 factor A is very much like that of vancomycin. The chief difference noticeable by inspection is the absence of the N—$CH_3$ resonance for N-(methyl)leucine in the spectrum of A51568. Thus, the proton nmr spectrum of vancomycin contains a methyl singlet at 2.39 ppm., which is absent from the proton nmr spectrum of antibiotic A51568 factor A.

Based on the molecular weight, the proton nuclear magnetic resonance spectrum data, and the elemental analyses, an empirical formula of $C_{65}H_{73}Cl_2N_9O_{24}$ is assigned to antibiotic A51568 factor A.

Potentiometric titration of antibiotic A51568 factor A in 66 percent aqueous dimethylformamide indicated pKa values of N6.2, 8.8, 10.3, and 12.85 (initial pH=6.12).

Antibiotic A51568 factor A has the following specific rotation: $[\alpha]_D^{25°C} = -20.4°$ (C = 10 mg/ml, water), $[\alpha]_{365}^{25°C} = -33.6°$ (C = 10 mg/ml, water).

The infrared absorption spectrum of antibiotic A51568 factor A in KBr pellet is shown in the accompanying drawing as Figure 1. The following distinguishable absorption maxima are observed: 3400 (broad, strong), 3380 (broad, strong), 1657 (broad, strong), 1587 (medium strong), 1505 (medium strong), 1424 (medium), 1396 (medium), 1328 (broad, weak), 1310 (broad, weak), 1231 (medium strong), 1174 (weak), 1156 (weak), 1128 (medium), 1062 (medium strong), 1028 (weak), 1015 (weak), 990 (weak), 882 (weak), 881 (weak) $cm^{-1}$.

The ultraviolet absorption maxima of antibiotic A51568 factor A in water under acid, neutral, and basic conditions are recorded in Table 2, which follows.

Table 2

UV Spectrophotometry of Antibiotic A51568 factor A

| Acid or Neutral max nm (ε) | Basic max nm (ε) |
|---|---|
| 278 (5,500) | 302 (6,000) |
| 234 (shoulder)(25,000) | 264 (shoulder) (10,000) |

Antibiotic A51568 factor B (formula I wherein n is z) is a white, amorphous solid. A51568 factor B has a molecular weight of about 1437, as determined by fast-atom-bombardment mass spectrometry.

The proton nuclear magnetic resonance spectrum of antibiotic A51568 factor B was determined in dimethylsulfoxide at 60°C and 360 MHz. The several six-membered rings of the structural formula are identified by letters of the alphabet, as indicated in the following formula:

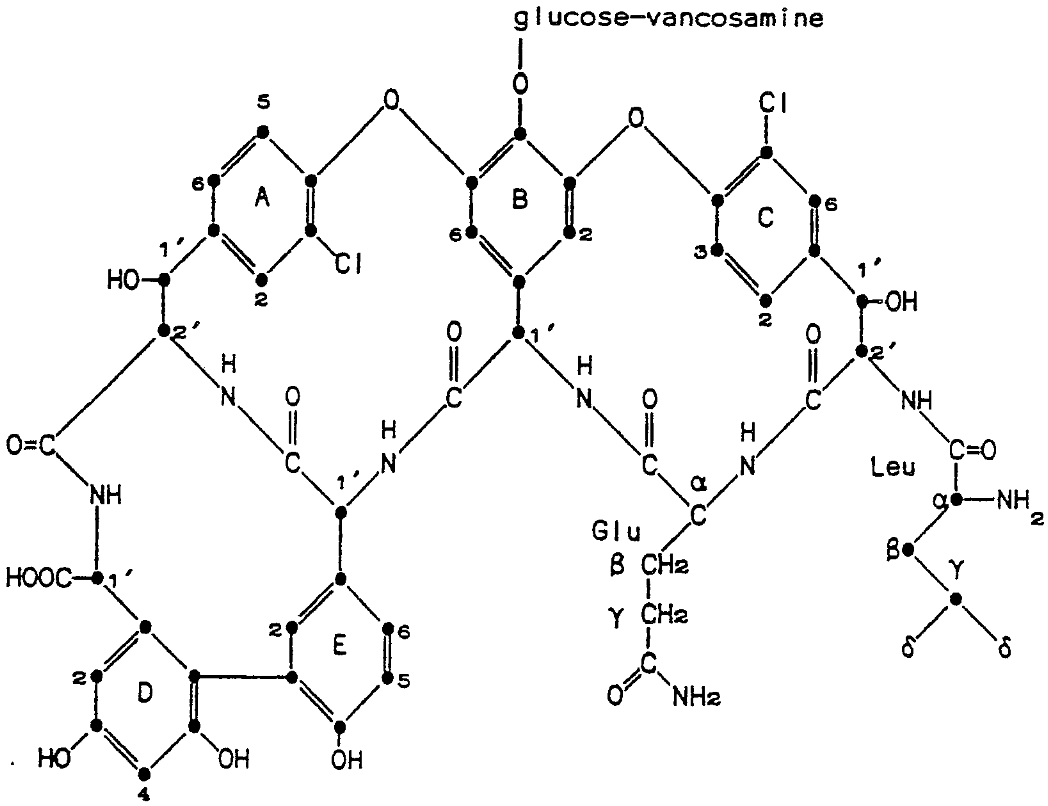

The table of Chemical Shifts, in ppm, follows in Table 3.

Table 3

Chemical Shifts for A51568 factor B (60°C)

| Assignment | | Assignment | |
|---|---|---|---|
| Ring A | Chem. Shift | Glutamine | Chem. Shift |
| -NH | 6.54 | -NH | 6.83 |
| -2' | 4.19 | -α | 4.06 |
| -1' | 5.13 | -β | 1.67 & |
| -OH | 5.85 | γ | 1.87 |
| -2 | 7.84 | $-NH_2$ | 6.83 & |
| -5 | 7.29 | | 6.41 |
| -6 | 7.47 | | |
| | | Leucine | |
| Ring B | | $-NH_2$ | ____* |
| -NH | 7.63 | -α | 4.05 |
| -1' | 5.67 | -β's | 1.63 |
| -2 | 5.60 | | 1.68 |
| -6 | 5.23 | -γ | 1.67 |
| | | -δ's | 0.92 |
| | | | 0.91 |

Table 3 cont'd.

Chemical Shifts for A51568 factor A

| Assignment | | Assignment | |
|---|---|---|---|
| Ring C | Chem. Shift | Vancosamine | Chem. Shift |
| -NH | ___* | -1 | 5.33 |
| -2' | 4.81 | -2 | 5.52 |
| -1' | 5.13 | -3 | 3.48 |
| -OH | 5.83 | -4 | 3.29 |
| -2 | 7.84 | -5 | 3.16 |
| -3 | 7.16 | -6 | 3.70 & |
| -6 | 7.13 | | 3.52 |
| Ring D | | Vancosamine | |
| -NH | 8.42 | -1 | 5.30 |
| -1' | 4.48 | -2 | 1.92 & |
| -2 | 6.29 | | 1.76 |
| -4 | 6.42 | ($3-CH_3$) | 1.37 |
| Ring E | | | |
| -NH | 8.48 | 4 | 3.16 |
| -1' | 4.48 | 5 | 4.67 |
| -2 | 7.18 | ($5-CH_3$) | 1.09 |
| -5 | 6.72 | | |
| -6 | 6.79 | | |

*Not assigned

The proton nuclear magnetic resonance spectrum of antibiotic A51568 factor B is very much like that of vancomycin and that of A51568 factor A. The chief differences noticeable by inspection are the absence of the N—$CH_3$ resonance for N-(methyl)leucine found in the spectrum of Vancomycin, and the absence of the asparagine resonances found in the spectra of Vancomycin and A51568 factor A. The asparagine resonances found in Vancomycin and in A51568 factor A are replaced by glutamine resonances in A51568 factor B.

Based on the mass spectral data, the proton nuclear magnetic resonance spectrum data, and comparison of this data to the corresponding data for A51568 factor A, an empirical formula of $C_{65}H_{73}Cl_2N_9O_{24}$ is assigned to antibiotic A51568 factor B.

A51568 antibiotic mixture containing factors A and B is produced by culturing a hitherto undescribed strain of *Nocardia orientalis* NRRL 15232.

This invention further relates to the hitherto undescribed strain of *Nocardia orientalis* NRRL 15232, and to biologically pure cultures thereof. For convenience, the biologically pure culture of *Nocardia orientalis* NRRL 15232 has been designated in our laboratory as culture A51568.1.

Culture A51568.1 is a variant strain derived through natural selection from culture A51568, which latter culture was initially isolated from a soil sample collected in Yucatan, Mexico.

Culture A51568.1 is classified as a strain of *Nocardia orientalis* based on simultaneous laboratory comparisons, as well as comparison with the published descriptions of *Nocardia* by D. Berd, "Laboratory Identification of Clinically Important Aerobic Actinomycetes", *Appl. Microbiol. 25(4),* 665—681 (1973); by Bergey's Manual of Determinative Bacteriology, 8th Edition, edited by R. E. Buchanan and N. E. Gibbons (The Williams and Wilkins Co., Baltimore); by R. E. Gordon *et al., "Nocardia coeliaca, Nocardia autotrophia,* and the Nocardin Strain", *Int. J. Syst. Bacteriol. 24(1),* 54—63 (1974); by R. E. Gordon *et al.,* "Resistance to Rifampin and Lysozyme of Strains of Some Species of *Mycobacterium* and *Nocardia* as a Taxonomic Tool", *Int. J. Syst. Bacteriol. 27(3),* 176—178 (1977); by S. A. Waksman, The Actinomycetes Vol. II, [The Williams and Wilkins Co., Baltimore (1961)]; and with the published descriptions of this species by R. E. Gordon *et*

*al.*, "Some Bits and Pieces of the Genus *Nocardia: N. carnea, N. vacinii, N. transvalensis, N. orientalis,* and *N. aerocolonigenes", J. Gen. Microbiol. 109,* 69—78 (1978); by S. J. Mishra *et al., "Identification of Nocardiae and Streptomycetes of Medical Importance", J. Clin. Microbiol. 11(6), 728—736 (1980); and by Pittenger et al., "Streptomyces orientalis,* n. sp., the Source of Vancomycin", *Antibiot. and Chemoth. VI(11),* 642—647 (1956).

The methods and media recommended for the International Streptomyces Project (ISP) for the characterization of *Streptomyces* species, as published by Shirling and Gottlieb ["Methods of Characterization of *Streptomyces* Species", *Int. J. Syst. Bacteriol. 16(3),* 313—340 (1966)], as well as methods recommended for the characterization of *Nocardia* species, as published by Gordon *et al., "Nocardia coeliaca, Nocardia autotrophia,* and the Nocardin Strain", *Int. J. Syst. Bacteriol. 24(1),* 54—63 (1974) were followed.

Characterization of A51568.1 Culture

Morphology

Culture A51568.1 produces an extensive substrate and aerial mycelium. When viewed under the light microscope, the aerial hyphae have a cobweb appearance. When grown under submerged, shaken conditions, the hyphae break into short mycelial fragments.

No conidia were observed when aerial hyphae were viewed by light microscopy. However, conidia are seen when studied by scanning electron microscopy. These features are observed when culture A51568.1 is grown on ISP medium No. 5 and tap water agar. The spore surface ornamentation is smooth (Sm). The spore shape is spherical to cylindrical, and the spores are poorly and irregularly formed. This morphology is classified in the nonstreptomycetes section as described in Bergey's Manual, *supra.*

Cultural Characteristics

Culture A51568.1 grew well on yeast dextrose agar, as well as on many other agar media, the growth being often wrinkled and flaky. Abundant aerial mycelia were produced having a spore mass color in the gray (GY) and sometimes white (W) color series. The nearest matching color tab for the gray color series in the Tresner and Backus system [see Tresner and Backus, "System of Color Wheels for Streptomycete Taxonomy", *Appl. Microbiol. 11,* 335—338 (1956)] is *d* light Gray to *2dc* yellowish Gray.

The aerial growth is neither as dense nor as long as typical *Streptomyces.* The aerial growth is produced on inorganic salts-starch agar (ISP No. 4), on glycerol-asparagine agar (ISP No. 5), and glycerol-glycine agar, and is best observed when grown on yeast-malt extract agar (ISP No. 2).

A dark brown distinctive color is observed on the reverse side in the following agar media: ISP No. 2, ISP No. 5, tyrosine agar (ISP No. 7), and glycerol-glycine agar. This color is unaffected by pH. A light to reddish brown soluble pigment is also elaborated in the above-named media, as well as in Czapek's solution agar and in glucose-asparagine agar. The reverse color of culture A51568.1 in other media is yellow-brown.

When plated for variability, culture A51568.1 showed no mixture of colony types, and culture A51568.1 is therefore regarded as a stable isolate.

The cultural characteristics of culture A51568.1 on various media compared with the cultural characteristics of *Nocardia orientalis* ATCC 19795 are presented in Table 4, which follows.

Color names were assigned according to the ISCC—NBS Centroid Color Charts Sample No. 2106 (National Bureau of Standards, U.S. Department of Commerce, 1958), and the Color Harmony Manual, 4th Edition (Color Standards Department, Container Corporation of America, Chicago, Illinois, 1958).

Table 4

Growth Characteristics of A51568.1 and ATCC 19795 on Various Media

| Medium | | A51568.1 | ATCC 19795 |
|---|---|---|---|
| ISP 2 | G: | Abundant-wrinkled surface | Abundant-wrinkled surface |
| | R: | 59.d.Br (no pH change) | 72.d.OY |
| | Am: | Abundant:d l. Gray (GY) | Abundant:2ba pale Yellow (Y) |
| | Sp: | Reddish-brown | None |
| ISP 3 | G: | Abundant | Abundant |
| | R: | 89.p.Y | 89.p.Y |
| | Am: | Good:d l.Gray (GY) | Good:d l.Gray (GY) |
| | Sp: | None | None |
| ISP 4 | G: | Abundant | Abundant |
| | R: | 72.d.OY | 70.l.OY |
| | Am: | Abundant:b Oyster White (W) | Abundant:b Oyster White (W) |
| | Sp: | None | None |
| ISP 5 | G: | Abundant | Abundant |
| | R: | 59.d.Br (no pH change) | 70.l.OY |
| | Am: | Abundant:b Oyster White (W) | Abundant:b Oyster White (W) |
| | Sp: | Light Brown | None |
| ISP 7 | G: | Abundant | Abundant |
| | R: | 65.br.Black (no pH change) | 71.m.OY |
| | Am: | Abundant:2ba p. Yellow (Y) | Abundant:2ba p. Yellow (Y) |
| | Sp: | Dark reddish-brown | None |
| Czapeks Solution | G: | Good | Good |
| | R: | 74.s.yBR | 70.l.OY |
| | Am: | Good:b Oyster White (W) | Good:b Oyster White (W) |
| | Sp: | Light brown | None |
| Emerson's Agar | G: | Abundant-wrinkled, flaky | Abundant-wrinkled, flaky |
| | R: | 74.s.yBr | 74.s.yBr |
| | Am: | Good:d.l.GYT 2ba p.Y (GY) | Good:2ba p. Yellow (Y) |
| | Sp: | None | None |

Table 4, continued

| | | | |
|---|---|---|---|
| | G: | Abundant | Abundant |
| Glucose Asparagine | R: | 75. deep γBr | 67. brill.OY |
| | Am: | Abundant:2dc y Gray (GY) | Abundant:2ba p. Yellow (Y) |
| | Sp: | Olive-brown | None |
| | G: | Abundant | Abundant |
| Glycerol-glycine | R: | 78.d.γB (no pH change) | 71.m.OY |
| | Am: | Abundant:2dc y Gray (GY) | Abundant:2ba p. Yellow (Y) |
| | Sp: | Olive-brown | None |
| | G: | Good | Good |
| Nutrient | R: | 70.1.OY | 70.1.OY |
| Agar | Am: | Good:b Oyster White (W) | Good:b Oyster White (W) |
| | Sp: | None | None |
| | G: | Fair | Fair |
| Tap | R: | 93.γ Gray | 93. γ Gray |
| $H_2O$ | Am: | Poor | Poor |
| | Sp: | None | None |
| | G: | Abundant-wrinkled, flaky | Abundant-slightly wrinkled |
| Yeast | R: | 68.s.OY | 68.s.OY |
| Dextrose | Am: | Abundant:d light Gray (GY) | Abundant:b Oyster White (W) |
| | Sp: | None | None |

G = growth R = reverse Am = aerial mycelia Sp = soluble pigment

Cell Wall Studies

Using hydrolyzed whole cells of A51568.1 culture, the presence of certain diagnostic sugars was determined by the chromatographic method of M. P. Lechevalier, "Identification of Aerobic Actinomycetes of Clinical Importance", *J. Lab. Clin. Med. 71,* 931—944 (1968).

Hydrolyzed whole cells were used to determine the isomers of diaminopimelic acid according to the method of Becker *et al., Appl. Microbiol. 11,* 421—423 (1964).

The results of these studies are set forth below.

| Test | Result Observed |
|---|---|
| Diagnostic sugars | Galactose, Arabinose |
| Isomers of 2,6-diaminopimelic acid | Ribose Meso-isomer |

These results represent a Type A whole-cell sugar pattern, and a Type IV cell wall, which combination of major cell wall constituents is indicative of the genus *Nocardia.* See M. P. Lechevalier, *supra.*

Using thin-layer chromatography of whole-cell methanolysates, according to the method described by

D. E. Minnikin *et al.*, "Differentiation of *Mycobacterium, Nocardia,* and Related Taxa by Thin-layer Chromatographic analysis of Whole-organism Methanolysates," *J. Gen. Microbiol. 88,* 200—204 (1975), no mycolic acid methyl esters were observed.

In addition to the above determinations, culture A51568.1 was studied and compared with published data on 21 *N. orientalis* reference strains, as well as simultaneous comparisons with NRRL 2451, NRRL 2452, and ATCC 19795. The comparison of the cultural, morphological, and physiological properties is recorded in Table 5, which follows.

Table 5

Comparison of Physiological Properties of A51568.1 and *N. orientalis* Reference Strains

| Characteristic | A51568.1 | NRRL 2451 | NRRL 2452 | ATCC 19795 | *N. orientalis* |
|---|---|---|---|---|---|
| Acid-fastness | - | - | - | - | - |
| Aerial hyphae | + | + | + | + | + |
| Catalase | + | + | + | + | + |
| Cell wall type | IV | IV | IV | IV | IV |
| Conidia | + | + | + | + | + |
| Decomposition of | | | | | |
| adenine | - | - | - | - | - |
| casein | + | + | + | + | + |
| DNA | + | + | + | + | ND |
| hypoxanthine | + | + | + | + | + |
| tyrosine | + | + | + | + | + |
| urea | + | + | + | + | + |
| xanthine | + | + | + | - | - |
| Fragmentation | + | + | + | + | + |
| Galatin liquefaction | + | + | + | + | ND |
| Gram Stain | + | + | + | + | ND |
| Hydrolysis of: | | | | | |
| esculin | + | + | + | + | + |
| hippurate | + | + | + | + | + |
| skim milk | + | + | + | + | + |
| starch | + | + | + | + | + |

Table 5 con't.

| Characteristic | A51568.1 | NRRL 2451 | NRRL 2452 | ATCC 19795 | *N. orientalis* |
|---|---|---|---|---|---|
| Melanoid pigmentation | - | - | - | - | ND |
| Morphology - cobweb | + | + | + | + | + |
| NaCl tolerance-% | 8 | 8 | 8 | 10 | ND |
| Nitrite from nitrate | - | - | - | + | - |
| Phosphatase | + | + | + | + | ND |
| Resistance to | | | | | |
| bacitracin | - | - | - | - | ND |
| lysozyme | + | + | + | + | - |
| novobiocin | - | - | - | + | ND |
| penicillin G | + | + | + | + | ND |
| rifampin | + | + | + | + | + |
| Spore color | GY-W | W-Y | W-Y | W-Y | W-Y |
| Spore shape spherical to cylindrical | + | + | + | + | + |
| Spore surface, smooth | + | + | + | + | + |
| Survival at 50°C, 8h | + | + | + | + | + |
| Temperature range, °C | 10-40 | 15-40 | 15-40 | 10-40 | 10-40 |
| Type A sugar pattern | + | + | + | + | + |
| Urease production | + | + | + | + | + |
| Utilization pattern of carbon compounds | + | + | + | + | + |
| Utilization pattern of organic acids | + | + | + | + | + |
| Vegetative growth optimum in the same medium | + | + | + | + | ND |

ND = not done

Further, the acid production from carbohydrates by cultures A51568.1, NRRL 2451, NRRL 2452, and ATCC 19795 was compared with the published results of 21 *N. orientalis* reference strains. This information is recorded in Table 6, which follows.

Table 6

Acid Production From Carbohydrates by Cultures A51568.1, NRRL 2451, NRRL 2452, ATCC 19795, and *N. orientalis* Reference Strains

| Characteristic | A51568.1 | NRRL 2451 | NRRL 2452 | ATCC 19795 | *N. orientalis* |
|---|---|---|---|---|---|
| Adonitol | - | + | + | + | + |
| l(+) Arabinose | + | + | + | + | + |
| Cellobiose | + | + | + | + | + |
| Cellulose | - | - | - | - | ND |
| Dulcitol | - | - | - | - | ND |
| i-Erythritol | + | + | + | + | + |
| Fructose | + | + | + | + | ND |
| d(+) Galactose | + | + | + | + | ND |
| Glucose | + | + | + | + | + |
| Glycerol | + | + | + | + | + |
| i-Inositol | + | + | + | + | + |
| Inulin | + | + | + | + | ND |
| d(+) Lactose | + | + | + | + | + |
| d(+) Maltose | + | + | + | + | + |
| d(-) Mannitol | + | + | + | + | + |
| d(+) Mannose | + | + | + | + | + |
| d(+) Melezitose | + | + | + | - | - |
| d(+) Melibiose | - | - | - | - | - |
| α-Me-D-Glucoside | + | + | + | + | + |
| d(+) Raffinose | - | - | - | - | - |
| l(+) Rhamnose | - | - | - | + | - |
| Salicin | + | + | + | + | ND |
| d(-) Sorbitol | - | - | - | - | - |
| Sucrose | + | + | + | + | ND |
| d(+) Trehalose | + | + | + | + | + |
| d(+) Xylose | + | + | + | + | + |
| Control | - | - | - | - | - |

ND = not done

Using the cultural, morphological, and physiological characteristics determined for A51568.1, comparison was made with the published descriptions of 14 taxa of *Nocardia,* as published by R. E. Gordon *et al.,* "Some Bits and Pieces of the Genus *Nocardia: N. carnea, N. vaccinii, N. transvalensis, N. orientalis,* and *N. aerocolonigenes', J. Gen. Microbiol. 109,* 69—78 (1978); and by S. J. Mishra *et al.,* "Identification of Nocardia and Streptomyces of Medical Importance", *J. Clin. Microbiol. 11(6),* 728—736 (1980). In addition, simultaneous laboratory comparisons were made between culture A51568.1 and cultures NRRL 2451, NRRL 2452, and ATCC 19795.

Similarity coefficients were then calculated from the equation

$$S = [Ns^+ + Ns^-]/[Ns^+ + Ns^- + Nd] \times 100$$

where

$Ns^+$ = number of positive similarities
$Ns^-$ = number of negative similarities
Nd = number of dissimilarities (differences)

See: W. A. Kurylowicz *et al.,* "Numerical Taxonomy of Streptomycetes", Polish Medical Publishers, Warsaw (1975).

The properties used to calculate similarity coefficients were mainly physiological characteristics. Comparisons were made by measuring all the other strains against culture A51568.1. Thus, A51568.1 compared to itself was rated as 100. The comparisons are recorded in Table 7, which follows.

TABLE 7
Coefficient of Similarity
Between Culture A51568.1 and
Several Nocardia Reference Strains

| Culture | Coefficient |
|---|---|
| A51568.1 | 100 |
| NRRL 2451 | 95 |
| NRRL 2452 | 95 |
| ATCC 19795 | 86 |
| *N. orientalis* (composite of 21 strains) | 89 |
| *N. aerocolonigenes* | 76 |
| *N. hirsuta* | 68 |
| *N. autotrophia* | 65 |
| *N. dassonvillei* | 63 |
| *N. madurea* | 63 |
| *N. brasiliensis* | 61 |
| *N. caviae* | 61 |
| *N. transvalensis* | 57 |
| *N. amarae* | 53 |
| *N. vaccinii* | 50 |
| *N. asteroides* | 47 |
| *N. carnea* | 47 |
| *N. pelletieri* | 39 |

A comparison of the similarities and differences between culture A51568.1 and *N. orientalis* ATCC 19795 is set forth in Table 8, which follows.

Table 8

Similarities and Differences of Culture A51568.1 and culture ATCC 19795

| Similarities | Differences |
|---|---|
| Acid from carbohydrates | Cultural characteristics on some media: |
| Aerial hyphae | aerial mycelia color |
| Catalase production | reverse color |
| Cell wall type IV | soluble pigmentation |
| Conidia present | Decomposition of xanthine |
| Cultural characteristics | NaCl tolerance |
| Decomposition of: | Nitrate reduction |
| casein | Resistance to novobiocin |
| DNA | |
| hypoxanthine | |
| tyrosine | |
| Fragmentation of mycelium | |
| Gelatin liquefaction | |
| Hydrolysis of: | |
| esculin | |
| hippurate | |
| skim milk | |
| starch | |
| Inability to decompose adenine | |
| Melanoid pigments absent | |
| Morphology | |
| Phosphatase production | |

Table 8, continued

| Similarities | Differences |
|---|---|
| Resistance to: | |
| lysozyme | |
| penicillin | |
| rifampin | |
| Sensitivity to bacitracin | |
| Spore shape | |
| Spore surface ornamentation | |
| Staining reaction | |
| Sugar pattern of whole cell hydrolysates type A | |
| Survival at 50°C for 8 hrs. | |
| Temperature range (10-40°C) | |
| Urease production | |
| Utilization of organic compounds | |
| Vegetative growth in CSM medium | |

Culture A51568.1 has been deposited and made a part of the stock culture collection of the Northern Regional Research Center, U.S. Department of Agriculture, Agricultural Research Service, Peoria, Illinois 61604, from which it is available to the public under the number NRRL 15232.

Also a part of this invention are the pharmaceutically-acceptable, non-toxic salts of antibiotics A51568 factors A and B. "Pharmaceutically-acceptable" salts are salts in which the toxicity of the compound as a whole toward warm-blooded animals is not increased relative to the non-salt form. Representatives and suitable salts of antibiotic A51568 factors A and B include those acid addition salts formed by standard reaction with both organic and inorganic acids such as, for example, sulfuric, phosphoric, hydrochloric, acetic, succinic, citric, lactic, maleic, and fumaric; as well as those salts formed by the carboxyl group with strong bases such as sodium hydroxide, potassium hydroxide, sodium carbonate, potassium carbonate, ammonium hydroxide, diethanolamine, and like bases.

The activity of antibiotic A51568 factor A against a variety of bacteria, as determined by the agar-dilution method, is reported in Table 9, which follows, wherein the minimum inhibitory concentration (MIC) values are recorded.

TABLE 9

Activity of A51568 Factor A
Against a Variety of Bacteria

| Bacteria | MIC (µg./ml.) |
|---|---|
| *Staphylococcus aureus* | |
| 3055* | 0.5 |
| X400 | 1 |
| V138 | 1 |
| V140 | 1 |
| V102 | 1 |
| *Staphylococcus epidermidis* | |
| 222 | 1 |
| 270 | 2 |
| 285 | 2 |
| 219 | 1 |
| 269 | 2 |
| *Streptococcus pyogenes* | |
| C203 | 0.5 |
| ATCC 10389 | 0.5 |
| *Streptococcus sp.* Group B | |
| 5 | 4 |
| 14 | 0.5 |
| *Streptococcus sp.* Group D | |
| X66 | 1 |
| 9960 | 4 |
| 2041 | 2 |
| 8043 | 1 |
| 9901 | 2 |
| *Streptococcus sp.* Group D | |
| 12253F | 1 |
| Mx161 | 1 |
| 2058 | 4 |
| *Streptococcus pneumoniae* | |
| Park I | 0.125 |
| BI—438 | 0.5 |
| *Haemophilus parainfluenzae* | |
| 7901 | 64 |
| 9796 | 64 |

TABLE 9 cont'd.

| Bacteria | MIC (µg./ml.) |
| --- | --- |
| *Haemophilus influenzae* | |
| C.L. | 32 |
| Mx366 | 32 |
| Mx371 | 64 |
| 76 | 64 |
| Bond | 64 |
| 16836 | 128 |
| 4842 | 32 |
| 312 | 128 |

*The name or number appearing under each organism identifies the strain of the organism employed in the test.

The *in vitro* activities of antibiotic A51568 factors A and B against a number of aerobic bacteria have been determined using a standard agar-dilution assay. The results, determined by reading the end point after 24 hours, are recorded in Table 10, which follows.

TABLE 10

Activity of A51568 Factors A and B Against Aerobic Bacteria

| Test Organism | MIC (µg./ml.) A | MIC (µg./ml.) B |
|---|---|---|
| *Staphylococcus aureus* 3055 (X1.1) | 1 | 2 |
| *Staphylococcus aureus* V41 | 1 | 2 |
| *Staphylococcus aureus* X400 | 2 | 2 |
| *Staphylococcus aureus* S13E | 1 | 2 |
| *Staphylococcus epidermidis* EPI1 | 2 | 2 |
| *Staphylococcus epidermidis* EPI2 | 1 | — |
| *Staphylococcus epidermidis* 222 | — | 2 |
| *Streptococcus pyogenes* C203 | 1 | 0.5 |
| *Streptococcus pneumoniae* Park I | 0.5 | 0.6 |
| *Streptococcus sp.* Group D X66 | 1 | 1 |
| *Streptococcus sp.* Group D 9960 | 4 | — |
| *Streptococcus sp.* Group D 2041 | — | 2 |
| *Haemophilus influenzae* (sensitive) Holt | 64 | — |
| *Haemophilus influenzae* (resistant) R252 | 128 | — |
| *Haemophilus influenzae* (sensitive) C.L. | — | 128 |
| *Haemophilus influenzae* (resistant) 76 | — | 64 |
| *Escherichia coli* N10 | >128 | >128 |
| *Escherichia coli* EC14 | >128 | >128 |
| *Escherichia coli* TEM | 128 | >128 |
| *Klebsiella pneumoniae* X26 | >128 | >128 |
| *Klebsiella pneumoniae* KAE | >128 | >128 |
| *Klebsiella pneumoniae* X6B | — | >128 |

Antibiotic A51568 factors A and B have been tested and found to be active against a number of anaerobic bacteria, as recorded in Table 11, which follows, the MIC values having been determined by the agar-dilution method.

TABLE 11

Activity of A51568 Factors A and B
Against Anaerobic Bacteria

| Test Organism | MIC (µg./ml.) A | MIC (µg./ml.) B |
|---|---|---|
| *Clostridium difficile* 2994 | 2 | 1 |
| *Clostridium perfringens* 81 | 1 | 1 |
| *Clostridium septicum* 1128 | 1 | 1 |
| *Eubacterium aerofaciens* 1235 | 2 | 1 |
| *Peptococcus asaccharolyticus* 1302 | 16 | 1 |
| *Peptococcus prevoti* 1281 | 32 | 4 |
| *Peptostreptococcus anaerobius* 1428 | 1 | 2 |
| *Peptostreptococcus intermedius* 1264 | 1 | 1 |
| *Propionibacterium acnes* 79 | 1 | 2 |
| *Bacteroides fragilis* 111 | 32 | 32 |
| *Bacteroides fragilis* 1877 | 32 | 16 |
| *Bacteroides fragilis* 1936B | 32 | 32 |
| *Bacteroides thetaiotaomicron* 1438 | 32 | 32 |
| *Bacteroides melaninogenicus* 1856/28 | >128 | >128 |
| *Bacteroides melaninogenicus* 2736 | 16 | >128 |
| *Bacteroides vulgatis* 1211 | 32 | 4 |
| *Bacteroides corrodens* 1874 | 32 | 32 |
| *Fusobacterium symbiosum* 1470 | 2 | 32 |
| *Fusobacterium necrophorum* 6054A | 2 | 128 |

Antibiotic A51568 factor A is also active against a number of strains of *Clostridium difficile*, as determined by the agar-dilution method. The results of the tests are recorded in Table 12, which follows.

TABLE 12

Activity of Antibiotic A51568 Factor A
Against *Clostridium difficile* Strains

| *Clostridium difficile* | MIC (μg./ml.) |
|---|---|
| 8484 | 2 |
| 6890 | 2 |
| 2634 | 2 |
| 78 | 2 |
| A—194 | 2 |
| A—195 | 2 |
| A—196 | 2 |
| A—279 | 2 |
| A—280 | 2 |
| A—281 | 2 |
| WAL—2112 | 2 |
| WAL—3657 | 2 |
| WAL—4268 | 2 |
| 107B | 2 |
| 111F | 2 |
| 1153 | 2 |
| 3424—5B | 2 |
| 3816 | 2 |
| 3950D | 2 |

Antibiotic A51568 factors A and B have shown *in vivo* antimicrobial activity against experimental bacterial infections. When two doses of test compound were administered subcutaneously to mice in illustrative infections, the activity observed is measured as an $ED_{50}$ [effective dose in mg/kg to protect fifty percent of the test animals: See Warren Wick *et al., J. Bacteriol. 81,* 233—235 (1961)]. The $ED_{50}$ values observed for antibiotic A51568 factors A and B are as follows (in units of mg/kg × 2):

| | A | B |
|---|---|---|
| *Staph. aureus* 3055 | 1.79 | 2.5 |
| *Strep. pyrogenes* C203 | 3.03 | 7.94 |
| *Strep. pneumoniae* Park I | 2.71 | 2.5 |

In one of its aspects this invention provides a method for treating infections in man or animals which comprises administering to said man or animal a non-toxic antibiotic-effect dose of between about 25 mg and about 2,000 mg of either antibiotic A51568 factor A or A51568 factor B, or a pharmaceutically-acceptable salt thereof.

In the treatment of infections in man, the antibiotic is administered by the parenteral route, e.g., by i.m. injection, or i.v. infusion. For injection, the antibiotic, or a pharmaceutically-acceptable diluent at the

desired concentration and administered. Suitable diluents include, for example, Water-for-Injection, 0.9% saline, 5% dextrose, Ringer's solution, or other commonly employed diluent. For administration by i.v. infusion, the antibiotic or salt thereof can be made up in a physiological fluid or dilute nutrient at a suitable concentration; for example, at a concentration between about 5% and about 10%, and slowly infused with the fluid. Alternatively, the antibiotic may be administered by the "piggy-back" method.

The antibiotic, or a pharmaceutically-acceptable salt thereof can be made up in dosage unit formulations in hermetically sealed vials, sterile rubber-stoppered vials, sterile, rubber-stoppered vials, or in plastic pouches. Such unit dosage forms can contain excipients such as antioxidants, solubilizing agents, dispersing agents, buffers, and the like. One such dosage unit formulation comprises 100 mg of A51568 factor A or B, or a pharmaceutically-acceptable, non-toxic salt thereof, in a rubber (butyl rubber) stoppered vial. Another dosage unit formulation comprises 250 mg of antibiotic A51568 factor A or B, or a salt thereof, in a sterile, sealed vial. For i.v. infusion, a dosage unit formulation of this invention comprises 5 g of antibiotic A51568 factor A or B, or a pharmaceutically-acceptable salt thereof, in a plastic pouch.

When antibiotic A51568 factor A or B is used as an antibacterial agent, it may be administered either orally or parenterally. As will be appreciated by those skilled in the art, the A51568 factor A or B antibiotic is commonly administered together with a pharmaceutically-acceptable carrier or diluent. The dosage of A51568 factor A or B antibiotic will depend upon a variety of considerations, such as, for example, the nature and severity of the particular infections to be treated. Those skilled in the art will recognize that appropriate dosage ranges and/or dosage units for administration may be determined by considering the MIC and $ED_{50}$ values herein provided, together with factors such as the patient or host, and the infecting organism.

The A51568 factor A and B antibiotics are useful *inter alia* for suppressing the growth of *Staphylococcus, Streptococcus,* and *Propionibacterium acnes* organisms, and the antibiotic could therefore be used, for example, in the treatment of acne. A51568 factors A and B, in purified state, can be formulated in pharmaceutically-acceptable diluents such as isopropyl alcohol for application to the skin. Such solutions can be made up with antibiotic concentrations of from about 1 to about 15 percent weight per volume. Alternatively, the antibiotic can be made up into creams or lotions for application to the skin.

A51568 factor A and B are also useful for suppressing the growth of *Clostridium difficile* organisms, which cause pseudomembranous colitis in the intestine. A51568 factors A and B could therefore be used in the treatment of pseudomembranous colitis by the oral administration of an effective dose of the antibiotic or a pharmaceutically-acceptable, non-toxic salt thereof, prepared in a pharmaceutically-acceptable dosage form. For such use the A51568 factor A or B can be administered in gelatin capsules or in liquid suspension.

A51568 factors A and B are produced by culturing the previously undescribed microorganism *Nocardia orientalis* NRRL 15232, or an A51568-producing mutant or varient thereof, in a culture medium containing assimilable sources of carbon, nitrogen, and inorganic salts, under submerged aerobic fermentation conditions until a substantial level of antibiotic activity is produced. Most of the antibiotic activity is found in the broth, while minor amounts of antibiotic activity may be associated with the mycelia. The antibiotic is most readily separated from the fermentaiton mixture by removal of the mycelia, i.e., the biomass, by filtration. The antibiotic is then isolated from the filtered fermentation broth, preferably by column chromatography over a suitable adsorbent using an appropriate eluting agent.

Suitable adsorbents include carbon, anion and cation exchange resins, polyamide, carboxymethylcelluloses, highly porous copolymers of styrene and divinylbenzene such as Diaion HP—20, the Amberlite XAD resins, and the Duolite resins such as hydrophilic, insoluble, molecular-sieve chromatographic mediums made by cross-linking dextran, and also TSK Gels. The Diaion resins are a product of Mitsubishi Chemical Industries, Limited, Tokyo, Japan. The Amberlite XAD resins are produced by Rohm and Haas, Philadelphia, Pennsylvania. The Duolite resins are products of Diamond Shamrock, Redwood City, California. Sephadex resins are manufactured by Pharmacia Fine Chemicals AB, Uppsala, Sweden. The TSK Gels are available from E. Merck, Darmstadt, Germany, and from Bio-Rad, 2200 Wright Ave., Richmond, California, 94804.

A number of different media may be used with *Nocardia orientalis* NRRL 15232, to produce A51568 factors A and B. For economy in production, optimal yield, and ease of product isolation, however, certain culture media are preferred. These media should contain assimilable sources of carbon, nitrogen, and inorganic salts. Suitable carbon sources include potato dextrin, blackstrap molasses, glucose, sucrose, lactose, maltose, and starch. Optimum levels of carbon sources are from about 2 to about 5 percent by weight.

Preferred nitrogen sources include sodium glutamate, meat peptone, sodium nitrate, ammonium nitrate, ammonium sulfate, soybean grits, and yeast.

Essential trace elements necessary for the growth and development of the organism may occur as impurities in other constituents of the media in amounts sufficient to meet the growth and biosynthetic requirements of the organism. However, it may be beneficial to incorporate in the culture media additional soluble nutrient inorganic salts capable of yielding sodium, potassium, magnesium, calcium, ammonium, chloride, carbonate, phosphate, sulfate, nitrate and like ions.

It may be necessary to add small amounts (i.e., 0.2 ml/L) of an antifoam agent such as propyleneglycol to large-scale fermentation media if foaming becomes a problem.

Although small quantities of A51568 factors A and B can be obtained by shake-flask culture,

submerged aerobic fermentation in tanks is preferred for producing substantial quantities of the antibiotic mixture. For tank fermentation, it is preferable to use a vegetative inoculum. The vegetative inoculum is prepared by inoculating a small volume of culture medium with the spore form, or mycelial fragments, to obtain a fresh, actively growing culture of the organism. The vegative inoculum is then transferred to a larger tank where, after a suitable incubation time, the A51568 antibiotic mixture is produced in optimal yield.

An alternate method of providing inoculum for the vegetative medium consists of substituting a lyophilized pellet for the aqueous spore suspension. Lyophilized pellets are prepared in a manner known in the art. Preparation of the spore suspension for lyophilization is similar to the preparation of the aqueous spore suspension, except that sterile calf serum is substituted for sterile distilled water.

NRRL 15232 can be grown over a broad temprature range of from about 25 to about 37°C. Optimum production of the A51568 antibiotic mixture appears to occur at a temperature of about 32 to 34°C.

As is customary in aerobic submerged culture processes, steile air is dispersed through the culture medium. For efficient growth of the organism, the volume of the air used in tank production is in the range of from about 0.1 to about 0.25 volumes of air per volume of culture medium per minute (v/v/m), with from about 100 to about 300 RPM agitation. An optimum rate in a 165-liter vessel containing 100 liters of fermentation medium is about 0.15 v/v/m, with agitation provided by an impeller rotating at about 200 RPM.

Antibiotic activity is generally present after about 24 hours and remains present for at least 168 hours during the fermentation period. Peak antibiotic production ccurs at from about 90 hours to about 114 hours fermentation time.

To increase production of the antibiotic mixture, p-hydroxyphenylglycine can be added to the medium at a level of $5 \times 10^{-3}$M. Such addition was found in one experiment to increase productivity by 64%.

Productivity is also affected by phosphate levels. Enrichment of phosphate by as little as $3 \times 10^{-4}$M (.05 mg/ml) was found in one experiment to depress yields by 20%.

The antibiotic activity is present in the supernatant. To sample, a whole broth sample is centrifuged for about 15 minutes at 1000 × g, and the centrate removed for assay. The assay is done microbiologically by an agar well plate test employing *Micrococcus luteus* ATCC 9341.

Production of the A51568 antibiotic mixture can be monitored during the fermentation by either agar diffusion using *Bacillus subtilis* ATCC 6633, or a turbidimetric method using *Straphylococcus aureus* ATCC 9114.

Accordingly, another aspect of the invention is a method of producing an antibiotic of formula (I), as defined above, which comprise cultivating *Nocardia orientalis* NRRL 15232, or an A51568-producing mutant or variant thereof, in a culture medium containing assimilable sources of carbon, nitrogen, and inorganic salts under submerged aerobic fermentation conditions until a substantial amount of antibiotic activity is produced.

In order to illustrate more fully the operation of this invention, the following examples are provided.

## Example 1

Preparation of First Stage Inoculum

The following medium was prepared for use in the agar slant culture of *Nocardia orientalis* NRRL 15232:

| Ingredient | Amount (g/L) |
|---|---|
| Precooked oatmeal | 60.0 |
| Yeast | 2.5 |
| $K_2HPO_4$ | 1.0 |
| Czapek's mineral stock | 5.0 ml/L |
| Agar | 25.0 |
| Deionized water | q.s. to 1.0 liter |

Czapek's mineral stock is prepared from the following ingredients:

| Ingredient | Amount (g/100 ml) |
|---|---|
| KCl | 10.0 |
| $MgSO_4 \cdot 7H_2O$ | 10.0 |
| $FeSO_4 \cdot 7H_2O$ | 0.2 |
| Deionised water | q.s. to 100 ml. |

Pre-sterilization pH = 6.2. Adjusted to pH 7.3 with aqueous sodium hydroxide solution. Post-sterilization pH = 6.7.

Spores of *Nocardia orientalis* NRRL 15232 were inoculated on an agar slant made up of the above-identified ingredients, and the thus-inoculated slant was incubated for seven days at about 30°C. The mature slant culture was then covered with sterile distilled water and scraped with a sterile tool to loosen the spores and the mycelium. One milliliter of the resulting spore suspension was used to inoculate 50 ml of vegetative medium having the following composition:

| Ingredient | Amount (g/L) |
|---|---|
| Potato dextrin | 30.0 |
| Molasses, blackstrap | 20.0 |
| Bacto-peptone (Difco Laboratories) | 7.0 |
| L-Tyrosine | 1.0 |
| Deionized water | q.s. to 1.0 liter |

The mixture had pH = 5.5, which was adjusted to pH 7.0 with aqueous sodium hydroxide solution before sterilization. Post-sterilization pH = 6.1.

The vegetative inoculum was incubated in a 250-ml wide-mouth Erlenmeyer flask at about 30°C for about 48 hours on a shaker rotating through an arc two inches in diameter at 250 RPM. This incubated medium is used either to inoculate small fermenters (the inoculum being 0.8% per volume of medium) or to inoculate second stage flasks for the production of a larger volume of mycelium.

Fermentation of A51568.1

One hundred liters of a production medium was inoculated with 0.8% (800 ml) of incubated vegetative medium from above. The production medium had the following composition:

| Ingredient | Amount (g/L) |
|---|---|
| Polypropyleneglycol (2000) | 0.2 |
| Potato dextrin | 30.0 |
| Blackstrap molasses | 20.0 |
| Bacto-peptone (Difco Laboratories) | 7.0 |
| L-Tyrosine | 1.0 |
| Deionized water | q.s. to 100 liters |

The medium had pH 5.2, which was adjusted to 7.1 with aqueous 5N sodium hydroxide. The medium was sterilized at 121°C at 17—19 psi. for 45 minutes. After sterilization, the medium had pH 6.2.

The inoculated production medium was allowed to ferment in a 165-liter fermentation tank for about 114 hours at a temperature of 30°C. The fermentation medium was aerated with sterile air at a rate of 0.15 v/v/m, and was stirred with conventional agitators at about 200 RPM.

The antibiotic activity is present in the supernatant of the fermentation mixture. The presence of the antibiotic activity was checked by centrifuging a sample of the whole broth at 1000 × g, and decanting the

centrate for assay. The sample is diluted with pH 6.0 phosphate buffer, and assayed microbiologically in an agar-well plate test using as assay organism *Micrococcus luteus* ATCC 9341.

Example 2

Isolation of Antibiotic A51568 Mixture

To three liters of antibiotic A51568 whole fermentation broth, containing factors A and B in a ratio of about 95 to 5, there was added filter aid (Hyflo Supercel, a diatomaceous earth, Johns-Manville Corp.), and the mixture was filtered. The filtrate, which measured 2.7 liters and had a pH of 7.8, was applied to Diaion HP—20 resin (a highly porous, styrene-divinylbenzene copolymer in bead form, Mitsubishi Chemical Industries, Ltd., Tokyo, Japan) contained in a 3 × 25 cm chromatography column. The effluent was discarded, and the column was washed with 1 liter of water and 500 ml of 25 percent methanol in water. The antibiotic A51568 mixture was eluted from the column with two 500-ml portions of 50 percent methanol in water. Factors A and B are adsorbed on HP—20 in the same way and are eluted therefrom under the same conditions. Accordingly, no separation of the factors was effected in this step. The eluant fractions were assayed for antibiotic activity using *B. subtilis.* The more active fractions were concentrated to a small volume and lyophilized. Crude antibiotic A51568 mixture weighing 905 mg and containing factors A and B in a ratio of about 95 to 5, was obtained.

Example 3

Purification of Antibiotic A51568 Mixture

The crude antibiotic A51568 mixture from Example 2 above (905 mg) was dissolved in 50 ml of water and applied to Sephadex CM—25 ($NH_4^+$ cycle) (Pharmacia Fine Chemicals AB, Uppsala, Sweden) contained in a 1.7 × 44 cm chromatography column. The effluent was discarded and the column was washed with 250 ml of water. The column was then eluted with a convex gradient of ammonium bicarbonate ($H_2O \rightarrow$ 1M $NH_4HCO_3$; 100 ml mixing chamber). Fifty 25-ml fractions were collected and checked for antibiotic activity using a *B. subtilis* assay.

Active fractions, that is, those containing antibiotic activity, were pooled. Because factors A and B are adsorbed and eluted differently when Sephadex CM—25 is used, the fractions rich in factor A were not the same fractions that were rich in factor B. Because the amount of factor B present in the mixture was small compared to the amount of factor A, the fractions rich in factor B exhibited a relatively low antibiotic activity compared to those rich in factor A. The pooled fractions contained relatively little factor B. To desalify the product, the pooled fractions were applied to a 1.7 × 10 cm chromatography column of Diaion HP—20 resin previously equilibrated in water and the column was washed with 200 ml of water and eluted with 100 ml of 50 percent methanol in water. The methanol eluate was evaporated to dryness. The residue was dissolved in a small amount of water and acidified with 0.1N aqueous hydrochloric acid. The acidified mixture was then lyophilized. There was obtained 25 mg of purified antibiotic A51568 factor A.

To isolate factor B, the method of Example 3 is followed using an HPLC assay in place of the assay using *B. subtilis.* This is necessary because the latter assay does not distinguish between factors A and B. Using an HPLC assay, the fractions rich in factors A and B can be separately identified and separately pooled. The separate pools are desalified as in the last step of Example 3 to provide one product that is rich in factor A and a second product that is rich in factor B.

Example 4

Isolation Purification and Separation of A51568 Factors A and B

To separate the A51568 mixture of factors A and B from A51568 whole broth the whole broth was filtered through Hyflo Supercel. After adjusting the filtrate to pH 6.0—6.5 with 5N HCl, Ionac X—236 resin ($H^+$) was added (4 l of resin per 100 l of filtrate) and the mixture was stirred to allow adsorption of the activity. The supernatant was discarded and the resin was washed with water (10 resin volumes). After discarding the wash, the X—236 resin was loaded into a column which was eluted with 0.1N ammonium hydrochloride (5 column volumes). The 4 liter fractions were immediately neutralized with 5N HCl. Antibiotic activity was monitored by *S. aureus* and by HPLC. The fractions containing A51568 factors A and B were pooled and loaded onto a column of Diaion HP—20 resin to desalify the mixture. The effluent was discarded. The resin was then eluted with 5% isopropyl alcohol containing 0.1% $H_3PO_4$. Fractions of 500 ml were collected and monitored for antibiotic activity. Active fractions were combined, concentrated, and lyophilized. The resulting impure material contained about 95% A51568 factor A and 5% of A51568 factor B.

To further purify the product and to simultaneously separate factors A and B, the impure mixture of factors A and B was dissolved in water and applied to a column of CM-Sephadex C—25. The column was eluted with a linear gradient from water to 0.25N $NH_4HCO_3$. Fractions wre monitored by HPLC and those containing factor A were pooled separately from those containing factor B.

To remove the salt added in the previous step, each pool was applied to a column of Diaion HP—20 resin which was eluted with 5% isopropyl alcohol containing 0.1% $H_3PO_4$. The appropriate fractions for each column were pooled on the basis of the HPLC assay. The two pools were concentrated and lyophilized yielding substantially pure A51568 factor A and substantially pure A51568 factor B, respectively.

**Claims for the Contracting States: BE CH DE FR IT LI LU NL SE**

1. An isolated antibiotic of the formula (I):

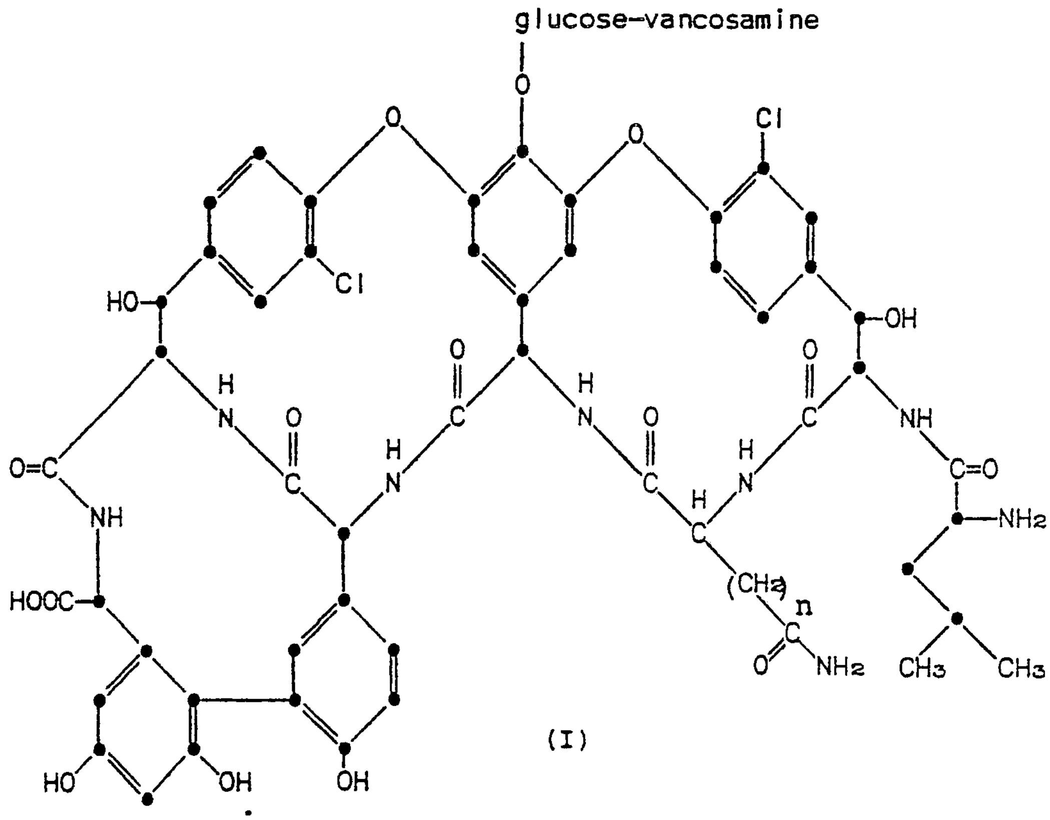

(I)

wherein n is 1 or 2, or a pharmaceutically acceptable salt thereof, or an antibiotic mixture comprising an antibiotic of formula (I) or a pharmaceutically acceptable salt thereof as the major component.

2. A method of producing an antibiotic or antibiotic mixture as defined in claim 1, which comprises cultivating *Nocardia orientalis* NRRL 15232, or a mutant or variant thereof capable of producing an antibiotic or antibiotic mixture as defined in claim 1, in a culture medium containing assimilable sources of carbon, nitrogen, and inorganic salts under submerged aerobic fermentation conditions until a substantial amount of antibiotic activity is prdouced.

3. The method of Claim 2 which includes the additional step of isolating an antibiotic of formula (I) as defined in claim 1 in which n is 1 from the culture medium.

4. The method of Claim 2 which includes the additional step of isolating an antibiotic of formula (I) as defined in claim 1 in which n is 2 from the culture medium.

5. *Nocardia orientalis* NRRL 15232.

6. A biologically pure culture of *Nocardia orientalis* NRRL 15232.

7. A pharmaceutical composition suitable for the treatment of bacterial infections in a mammal which comprises as the principal active ingredient an antibiotic compound of formula (I) as claimed in claim 1, or a pharmaceutically-acceptable, salt thereof, associated with one or more pharmaceutically-acceptable excipients on carriers therefor.

8. An antibiotic of formula (I) as defined in claim 1 for use as an antibiotic.

**Claims for the Contracting State: AT**

1. A method of producing an antibiotic of the formula (I):

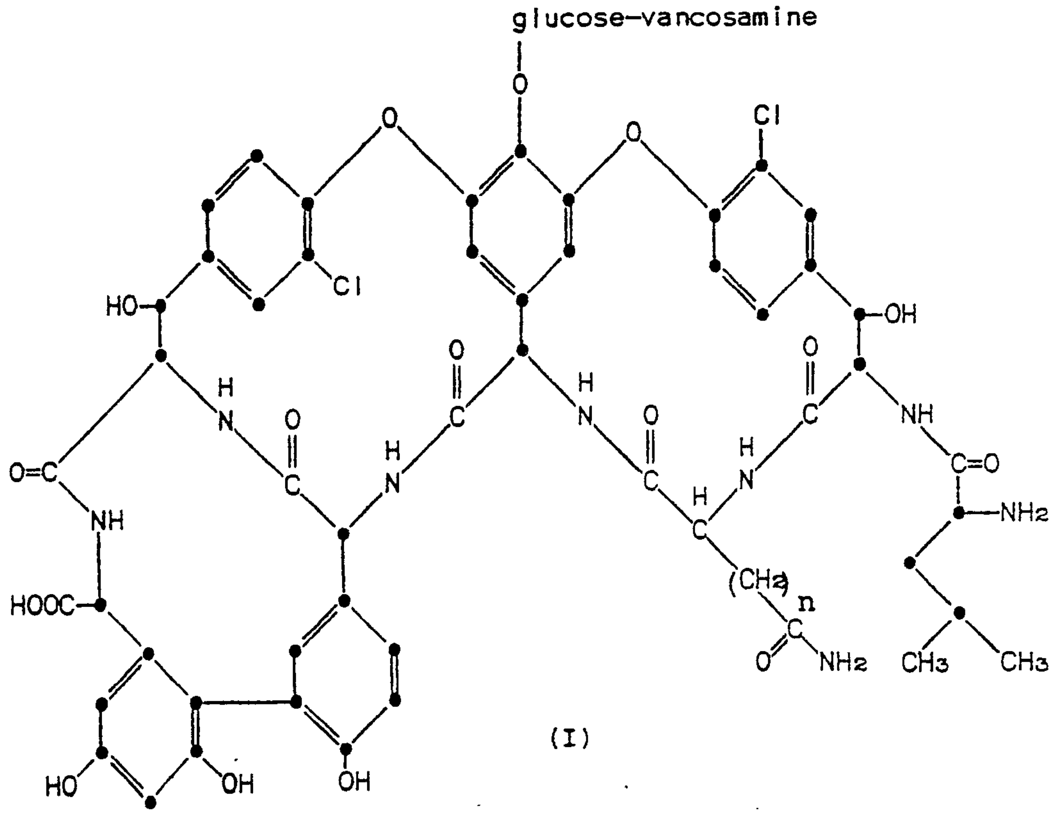

(I)

wherein n is 1 or 2, or a pharmaceutically acceptable salt thereof, or an antibiotic mixture comprising an antibiotic of formula (I) or a pharmaceutically acceptable salt thereof as the major component, which comprises cultivating *Nocardia orientalis* NRRL 15232, or a mutant or variant thereof capable of producing the said antibiotic or antibiotic mixture, in a culture medium containing assimilable sources of carbon, nitrogen, and inorganic salts under submerged aerobic fermentation conditions until a substantial amount of antibiotic activity is produced.

2. A method according to claim 1, which includes the additional step of isolating an antibiotic of formula (I) as defined in claim 1, in which n is 1, from the culture medium.

3. A method according to claim 1, which includes the additional step of isolating an antibiotic of formula (I) as defined in claim 1, in which n is 2, from the culture medium.

**Patentansprüche für die Vertragsstaaten: BE CH DE FR IT LI LU NL SE**

1. Isoliertes Antibiotikum der Formel (I)

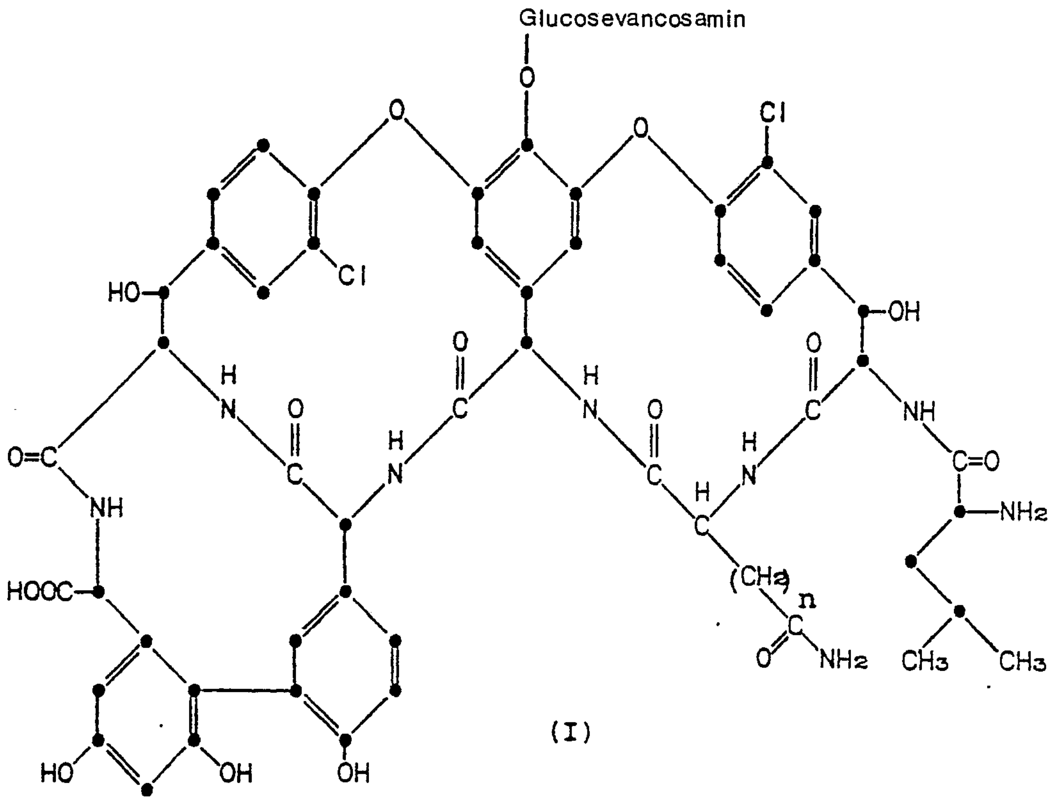

(I)

worin n für 1 oder 2 steht, oder ein pharmazeutisch annehmbares Salz hiervon oder ein antibiotisches Gemsich aus einem Antibiotikum der Formel (I) oder einen pharmazeutisch annehmbaren Salz hiervon als hauptsächliche Komponente.

2. Verfahren zur Herstellung eines Antibiotikums oder eines antibiotischen Gemisches nach Anspruch 1, dadurch gekennzeichnet, daß Nocardia orientalis NRRL 15 232 oder eine Mutante oder Variante hiervon, welche zur Bildung eines Antibiotikums oder eines antibiotischen Gemisches nach Anspruch 1 befähigt ist, in eine Kulturemedium, das assimilierbare Quellen für Kohlenstoff, Stickstoff und anorganische Salz enthält, unter submersen aeroben Fermentationsbedingungen so lange gezüchtet wird, bis eine wesentliche Menge an antibiotischer Aktivität gebildet ist.

3. Verfahren nach Anspruch 2, dadurch gekennzeichnet, daß das Antibiotikum der Formel (I) nach Anspruch 1, worin n für 1 steht, in einer weiteren Stufe aus dem Kulturmedium isoliert wird.

4. Verfahren nach Anspruch 2, dadurch gekennzeichnet, daß das Antibiotikum der Formel (I) nach Anspruch 1, worin n für 2 steht, in einer weiteren Stufe aus dem Kulturmedium isoliert wird.

5. Nocardia orientalis NRRL 15 232.

6. Biologisch reine Kultur von Nocardia orientalis NRRL 15 232.

7. Pharmazeutische Zusammensetzung, geeignet zur Behandlung von Bakterieninfektionen bei einem Säugetier, dadurch gekennzeichnet, daß sie als hauptsächlichen aktiven Bestandteil ein antibiotische Verbindung der Formel (I) nach Anspruch 1 oder ein pharmazeutisch annehmbares Salz hiervon in Verbindung mit einem oder mehreren pharmazeutisch annehmbaren Hilfsstoffen oder Trägern hierfür enthält.

8. Verwendung eines Antibiotikums der Formel (I) nach Anspruch 1 als antibiotisches Mittel.

**Patentansprüche für den Vertragsstaat: AT**

1. Verfahren zur Herstellung eines Antibiotikums der Formel (I)

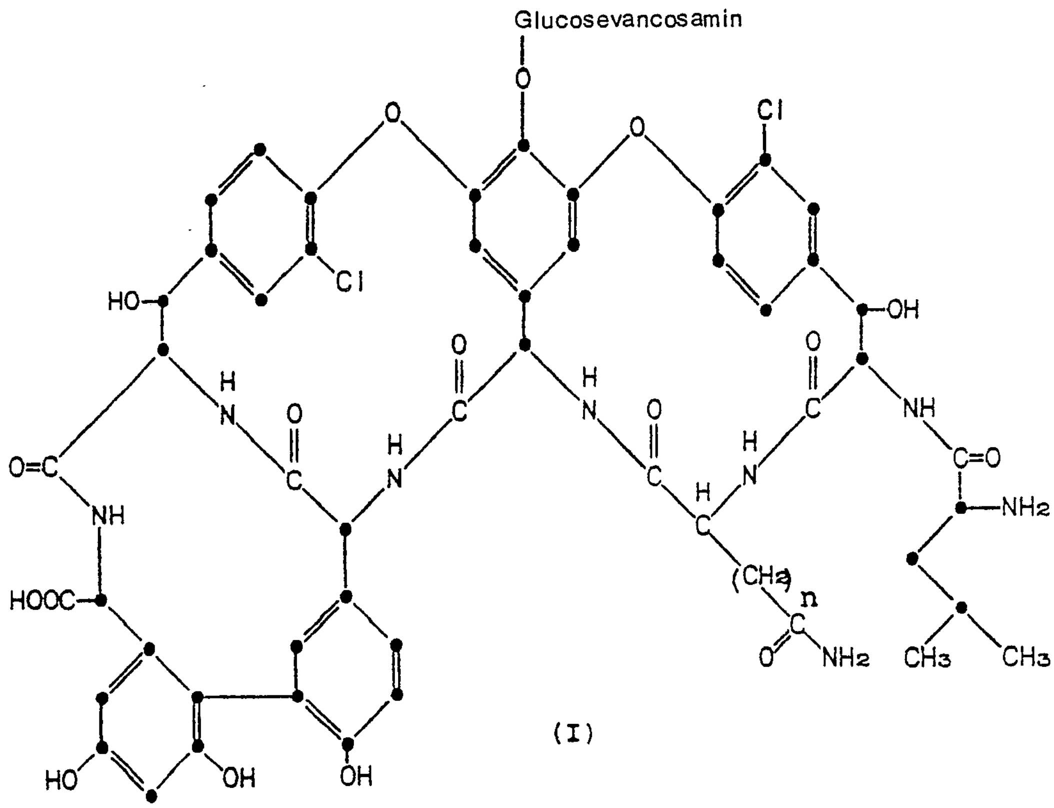

(I)

worin n für 1 oder 2 steht, oder eines pharmazeutisch annehmbaren Salzes hiervon oder eines antibiotischen Gemisches aus einem Antibiotikum der Formel (I) oder einem pharmazeutisch annehmbaren Salz hiervon als hauptsächliche Komponente, dadurch gekennzeichnet, daß Nocardia orientalis NRRL 15 232 oder eine Mutante oder Variante hiervon, welche zur Bildung eines Antibiotikums oder eines antibiotischen Gemisches befähigt ist, in einem Kulturmedium, das assimilierbare Quellen für Kohlenstoff, Stickstoff und anorganische Salze enthält, unter submersen aeroben Fermentationsbedingungen so lange gezüchtet wird, bis eine wesentliche Menge an antibiotischer Aktivität gebildet ist.

2. Verfahren nach Anspruch 1, dadurch gekennzeichnet, daß das Antibiotikum der Formel (I) nach Anspruch 1, worin n für 1 steht, in einer weiteren Stufe aus dem Kulturmedium isoliert wird.

3. Verfahren nach Anspruch 1, dadurch gekennzeichnet, daß das Antibiotikum der Formel (I) nach Anspruch 1, worin n für 2 steht, in einer weiteren Stufe aus dem Kulturmedium isoliert wird.

**Revendications pour les Etats contractants: BE CH DE FR IT LI LU NL SE**

1. Antibiotique isolé de formule (I):

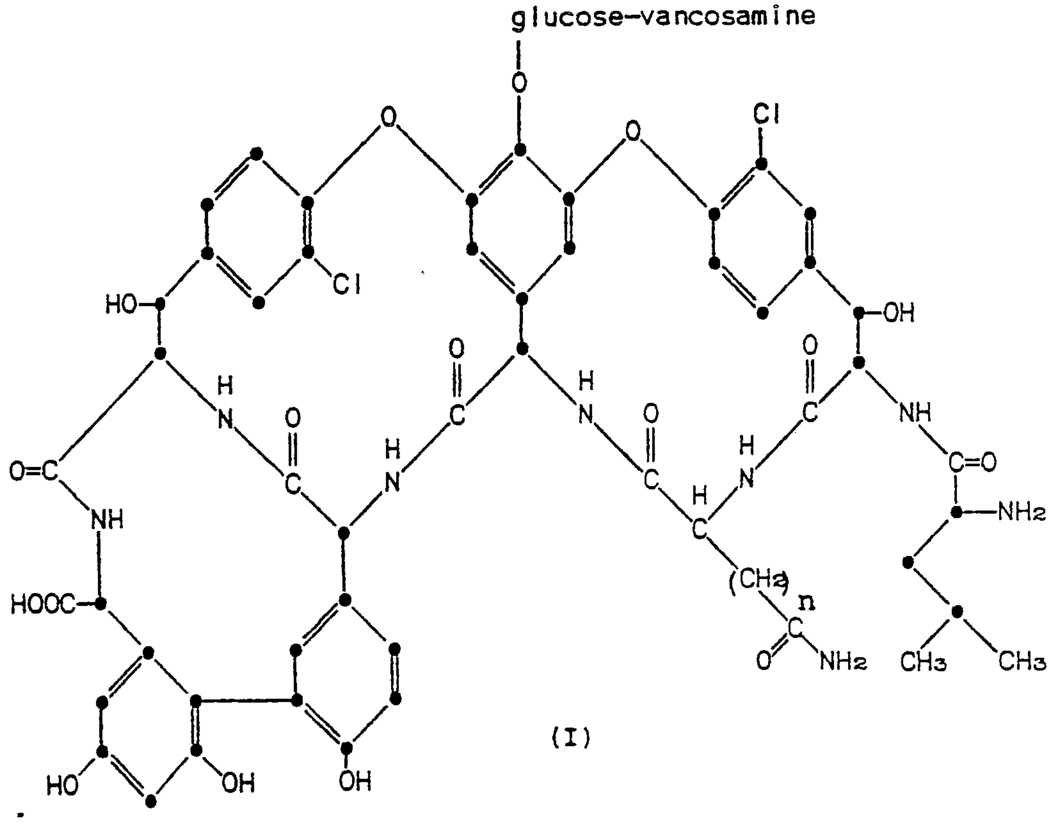

(I)

dans laquelle n est égal à 1 ou 2, ou de ses sels pharmaceutiquement acceptables, ou encore un mélange antibiotique comprenant un antibiotique de formule (I) ou de ses sels pharmaceutiquement acceptables comme composant principal.

2. Procédé de préparation d'un antibiotique ou d'un mélange d'antibiotiques comme défini dans la revendication 1, caractérisé en ce qu'il consiste à cultiver Nocardia orientalis NRRL 15232, ou un mutant ou un variant de celui-ci, capable de produire un antibiotique ou un mélange d'antibiotiques comme défini dans la revendication 1, dans un milieu de culture contenant des sources assimilables de carbone, d'azote et de sels inorganiques, dans des conditions de fermentation aérobie submergée, jusqu'à la production d'une importante quantité d'activité antibiotique.

3. Procédé selon la revendication 2, caractérisé en ce qu'il comprend l'étape supplémentaire consistant à isoler un antibiotique de formule (I) comme défini dans la revendication 1, où n est égal à 1, à partir du milieu de culture.

4. Procédé selon la revendication 2, caractérisé en ce qu'il comprend l'étape supplémentaire consistant. à isoler un antibiotique de formule (I) comme défini dans la revendication 1, dans laquelle n est égal à 2, du milieu de culture.

5. Nocardia orientalis NRRL 15232.

6. Culture biologiquement pure de Nocardia orientalis NRRL 15232.

7. Composition pharmaceutique appropriée pour le traitement d'infections bactériennes chez un mammifère, caractérisée en ce que, comme ingrédient actif principal, il contient un composé antibiotique de formule (I) selon la revendication 1, ou un de ses sels pharmaceutiquement acceptables, en association avec un ou plusieurs excipients pharmaceutiquement acceptables sur des supports pour cet ingrédient.

8. Antibiotique de formule (I) comme défini dans la revendication 1, en vue de l'utiliser comme antibiotique.

**Revendications pour l'Etat contractant: AT**

1. Procédé de préparation d'un antibiotique de formule (I):

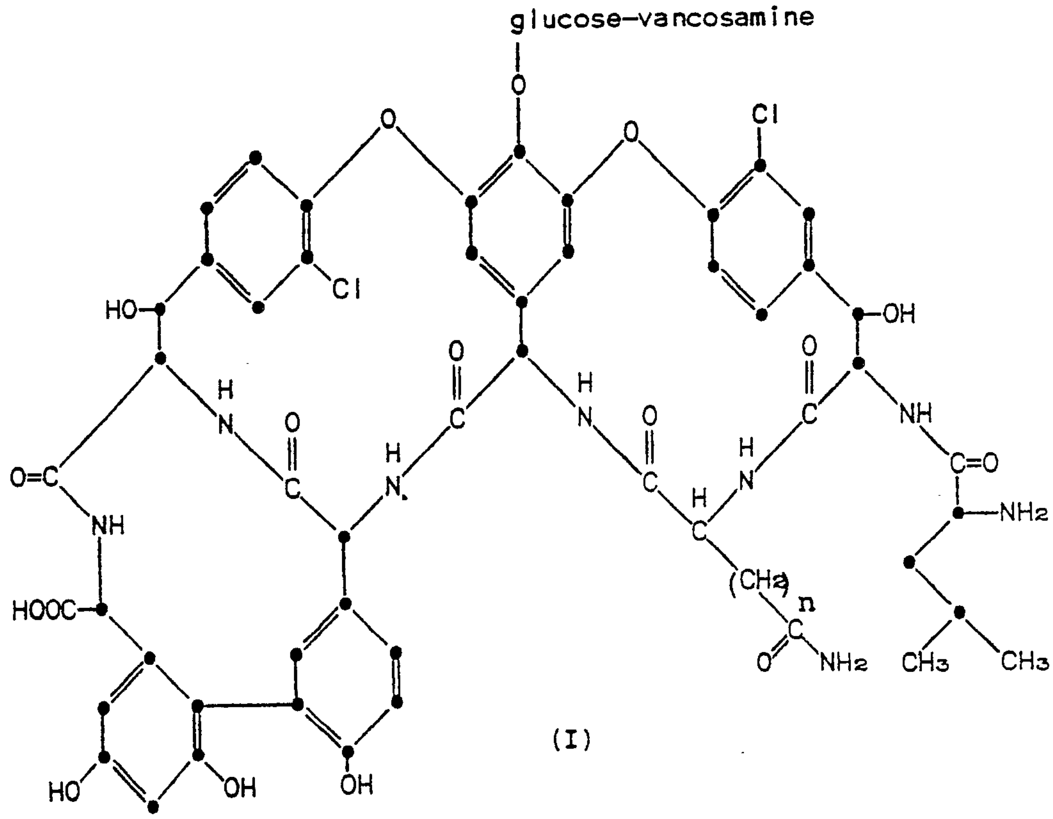

(I)

dans laquelle n est égal à 1 ou 2, ou d'un de ses sels pharmaceutiquement acceptables, ou encore d'un mélange d'antibiotiques comprenant un antibiotique de formule (I) ou un de ses sels pharmaceutiquement acceptables comme composant principal, caractérisé en ce qu'il consiste à cultiver Nocardia orientalis NRRL 15232, ou un de ses mutants ou variants, capable de produire cet antibiotique ou ce mélange d'antibiotiques, dans un milieu de culture contenant des sources assimilables de carbone, d'azote, et de sels inorganiques, dans des conditions de fermentation aérobie submergée jusqu'à la production d'un importante quantité de l'activité antibiotique.

2. Procédé selon la revendication 1, caractérisé en ce qu'il comprend l'étape supplémentaire consistant à isolier un antibiotique de formule (I) comme défini dans la revendication 1, dans laquelle n est égal à 1, du milieu de culture.

3. Procédé selon la revendication 1, caractérisé en ce qu'il comprend l'étape supplémentaire consistant à isolier un antibiotique de formule (I) comme défini dans la revendication 1, dans laquelle n est égal à 2, du milieu de culture.

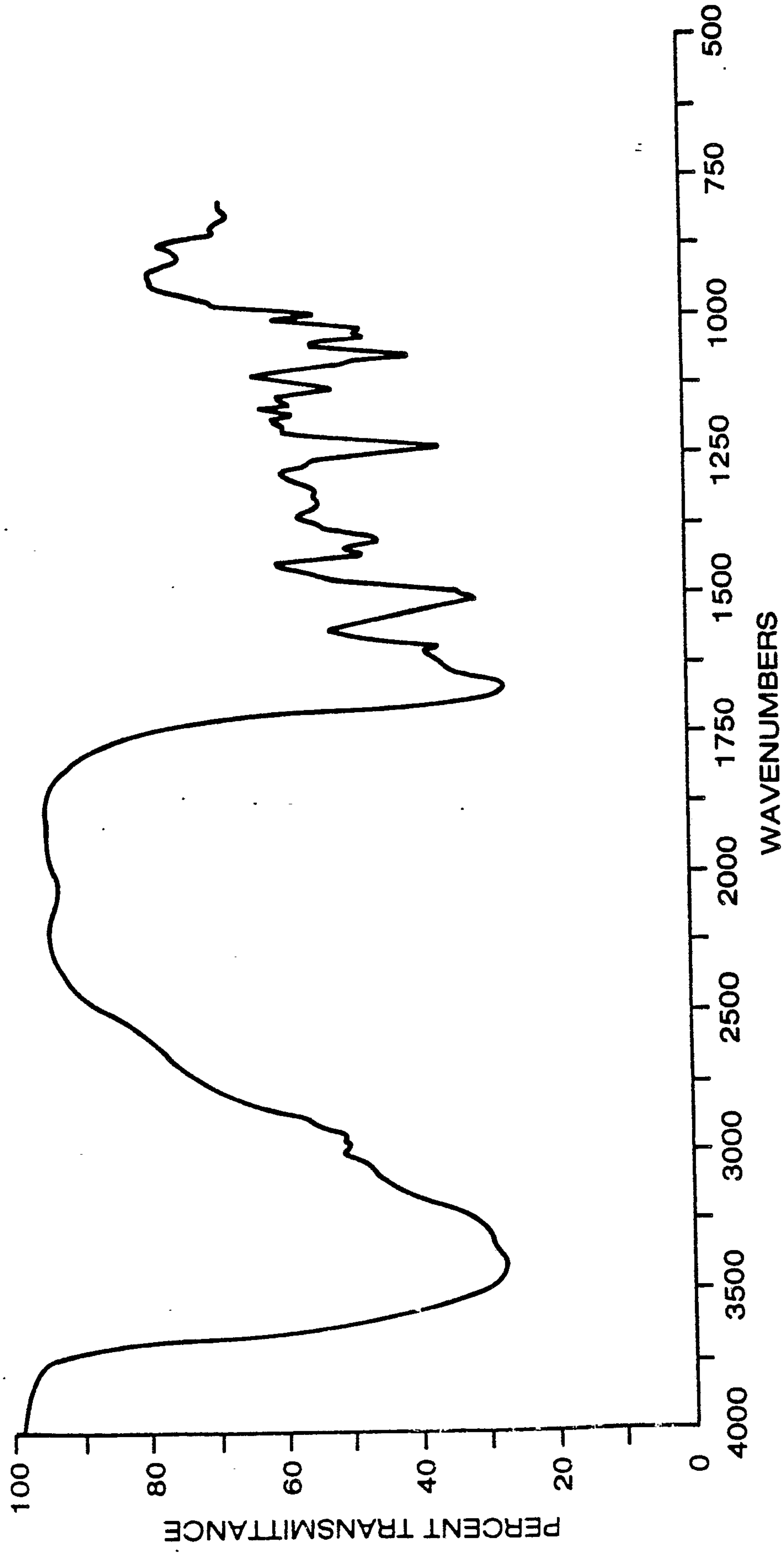
PERCENT TRANSMITTANCE
100
80
60
40
20
0
WAVENUMBERS
4000
3500
3000
2500
2000
1750
1500
1250
1000
750
500

FIG. I